# EUROPEAN PATENT APPLICATION

(11) **EP 4 427 800 A1**
(43) Date of publication of application: **11.09.2024**
(21) Application number: 22890339.9
(22) Date of filing: 02.11.2022
(51) Int. Cl.: A61N 1/40, A61N 1/06, A61N 1/32, A61N 1/08, A61N 1/02

(54) **RF ELECTRODE CARTRIDGE FOR SKIN COSMETIC TREATMENT COMPRISING RELAY, AND HANDPIECE FOR SKIN COSMETIC TREATMENT COMPRISING SAME**

(30) Priority: 04.11.2021 KR 20210150159
(71) Applicant: CLASSYS INC., Seoul 06220 (KR)
(72) Inventor: JEONG, Ha Gil, Seoul 08735 (KR); LEE, Seok Joo, Seoul 01781 (KR)
(74) Representative: Zimmermann, Tankred Klaus
(86) International application number: PCT/KR2022/016964
(87) International publication number: WO 2023/080608

(57) **Abstract**

The present invention relates to: an RF electrode cartridge for skin cosmetic treatment, the RF electrode cartridge comprising a relay; and a handpiece for skin cosmetic treatment, the handpiece comprising the RF electrode cartridge. The relay, which controls electrical signals applied to an RF electrode, is positioned in the RF electrode cartridge, and when the RF electrode cartridge is replaced, the relay, which is a consumable part, is also replaced, thus making it possible to use the handpiece without replacing a handpiece body casing, and reducing the hassle and cost of replacing the handpiece body casing.

## Description

### Technical Field

The present disclosure relates to an RF electrode cartridge including a relay for skin cosmetic treatment, and relates to a handpiece including the RF electrode cartridge for skin cosmetic treatment. More particularly, the present disclosure relates to an RF electrode cartridge including a relay for skin cosmetic treatment and being configured such that the relay is positioned in the RF electrode cartridge so that the useful life of a handpiece body casing is semi-permanently extended, and relates to a handpiece including the RF electrode cartridge for skin cosmetic treatment.

### Background Art

Generally, a handpiece provided with an RF electrode is a device configured to heat and treat skin and underlying tissues of the skin by using the RF (radio frequency) electrode.

The handpiece provided with the RF electrode is used for skin care treatment to restore the skin by increasing a temperature of the skin after a cryolipolysis procedure as an obesity treatment and to heat the skin and the underlying tissues of the skin to cause contraction of collagen or induce a wound healing reaction of the collagen.

Recently, due to increasing interest in skin cosmetic treatment, the handpiece provided with the RF electrode is used in a variety of ways for skin remodeling/resurfacing, for wrinkle removal, and for the treatment and so on of sebaceous glands, hair follicle adipose tissue, and spider veins, by the contraction or the wound healing reaction of the collagen in the lower dermis.

The handpiece provided with the RF electrode includes the RF electrode including an RF electrode body, and includes a handpiece body casing to which the RF electrode cartridge is coupled such that the RF electrode cartridge is capable of being detached from the handpiece body casing and which is connected to a control body.

The handpiece provided with the RF electrode controls an electrical signal applied to the RF electrode that is provided on a first surface of the RF electrode cartridge, so that various patterns are implemented according to the type of skin treatment, thereby increasing an effect on the corresponding skin treatment.

In addition, a relay for controlling the electrical signal applied to the RF electrode is provided in the handpiece body casing.

The RF electrode cartridge is a consumable part in which the number of uses is preset, and is replaced when the number of uses reaches the preset number of uses.

In addition, since the relay provided in the handpiece body casing is also a consumable part in which the number of uses is preset, so that the handpiece body casing is also replaced when the number of uses of the relay reaches the preset number of uses.

However, in the handpiece provided with the conventional RF electrode, since the number of uses of the RF electrode cartridge is preset and the number of uses of the relay is also preset, the RF electrode cartridge and the handpiece body casing are required to be replaced periodically, so that there is a problem that the maintenance cost according to the replacement cost is increased when the handpiece provided with the conventional RF electrode is used.

As related art related to the present disclosure, Korean Patent No. 10-2048384 'FLUID RF ELECTRODE ASSEMBLY FOR BEAUTY TREATMENT OF SKIN AND HANDPIECE FOR BEAUTY TREATMENT OF SKIN USING THE SAME' (registered on November 19, 2019) has been proposed.

### Disclosure

### Technical Problem

An objective of the present disclosure is to provide an RF electrode cartridge including a relay for skin cosmetic treatment and a handpiece including the RF electrode cartridge for skin cosmetic treatment, the RF electrode cartridge being configured such that the relay controlling an electrical signal applied to an RF electrode is positioned in the RF electrode cartridge so that the relay that is a consumable part is replaced when the RF electrode cartridge is replaced.

Another objective of the present disclosure is to provide an RF electrode cartridge including a relay for skin cosmetic treatment and a handpiece including the RF electrode cartridge for skin cosmetic treatment, the RF electrode cartridge being provided with a vibration motor unit such that pain that occurs during skin treatment is capable of being minimized and a treatment effect is capable of being increased.

Still another objective of the present disclosure is to provide an RF electrode cartridge including a relay for skin cosmetic treatment and a handpiece including the RF electrode cartridge for skin cosmetic treatment, the RF electrode cartridge being configured such that electric power is applied to an RF electrode body only when the RF electrode cartridge that is brought into contact with the skin, thereby being capable of realizing a safe and convenient treatment.

### Technical Solution

In order to achieve the objectives described above, according to an embodiment of the present disclosure, there is provided an RF electrode cartridge including a relay for skin cosmetic treatment, the RF electrode cartridge including: a cartridge casing; an RF electrode body positioned on a first surface of the cartridge casing, the RF electrode body being brought into contact with skin, and the RF electrode body being configured to apply RF energy inside the skin; the relay mounted in the cartridge casing and electrically connected to the RF electrode body, the relay being configured to control an operation of the RF electrode body; and a relay first connection terminal part for electrically connecting the relay to a control body.

In order to achieve the obj ectives described above, according to an embodiment of the present disclosure, there is provided a handpiece for skin cosmetic treatment, the handpiece including: an RF electrode cartridge including an RF electrode body being brought into contact with skin; and a handpiece body casing to which the RF electrode cartridge is coupled such that the RF electrode cartridge is capable of being detached from the handpiece body casing, the handpiece body casing being connected to a control body, wherein the RF electrode cartridge includes the embodiment of the RF electrode cartridge including the relay for skin cosmetic treatment.

### Advantageous Effects

In the present disclosure, since the relay that controls the electrical signal applied to the RF electrode is positioned in the RF electrode cartridge, the relay that is the consumable part is replaced when the RF electrode cartridge is replaced. Therefore, the handpiece body casing is capable of being used without being replaced, so that there is an effect that the hassle and the cost of replacing the handpiece body casing may be reduced.

In the present disclosure, since the vibration motor unit is provided, there are effects that pain that occurs during the skin treatment may be minimized and the treatment effect may be increased.

In the present disclosure, since electric power is applied to the RF electrode body only when the RF electrode cartridge that is brought into contact with the skin is pressed, there are effects that a safe and convenient treatment may be realized and safety during the treatment may be increased.

### Description of Drawings

FIG. 1 is a perspective view illustrating an embodiment of a handpiece for skin cosmetic treatment according to the present disclosure.
FIG. 2 is a perspective view illustrating an embodiment of an RF electrode cartridge including a relay for skin cosmetic treatment according to the present disclosure.
FIG. 3 is a cross-sectional view illustrating an embodiment of the RF electrode cartridge including the relay for skin cosmetic treatment according to the present disclosure.
FIG. 4 is an exploded perspective view illustrating an embodiment of a handpiece for skin cosmetic treatment according to the present disclosure.
FIG. 5 is a cross-sectional view illustrating an embodiment of the handpiece for skin cosmetic treatment according to the present disclosure.
FIG. 6 is a plan view illustrating the RF electrode cartridge for skin cosmetic treatment in an embodiment of the handpiece for skin cosmetic treatment according to the present disclosure.
FIG. 7 is a bottom view illustrating a handpiece body casing in an embodiment of the handpiece for skin cosmetic treatment according to the present disclosure.

### *DESCRIPTION OF MAIN REFERENCE NUMERALS OF DRAWINGS*

100: RF electrode cartridge 110: Cartridge casing
110a: First casing part 110b: Second casing part
111: Cooling space 112: Temperature sensor
113: Terminal insulation part 114: Cooling space cover part
114a: Ball coupling part 115: Electrode on/off switch
115a: Switch button 115b: Switch spring
116: Switch mounting protrusion part 120: RF electrode body
121: RF electrode member 121a: Electrode connection terminal
122: Insulation film member 130: Cartridge locking part
140: Ball joint part 150: Bellows member
150a: Pin insertion part 151: Bellows supporting frame member
152: Frame fixing pin 160: Vibration motor unit
170: First coolant line part 180: Second coolant line part
190: Relay 191: Electrode control substrate part
192: Relay first connection terminal part 200: Handpiece body casing
200a: Coolant circulation line part 200b: Cartridge insertion part
210: Cartridge coupling block 211: Relay second connection terminal part
212: Line connection part 220: Spring member for a cartridge
[46] 230: Cartridge movement guide part 231: Movement guide bar part
232: Movement guide block 240: Protrusion insertion part
241: Protrusion part for pressing a switch

### Mode for Invention

Hereinbelow, the present disclosure will be described in more detail.

An exemplary embodiment of the present disclosure will be described in detail with reference to the accompanying drawings. Prior to the detailed description of the present disclosure, it should be noted that the terms and words used in the specification and the claims should not be construed as being limited to ordinary meanings or dictionary definitions. Therefore, the description proposed herein is just an exemplary embodiment for the purpose of illustrations only, not intended to limit the scope of the present disclosure, so it should be understood that other equivalents and modifications could be made thereto without departing from the scope of the present disclosure at the time at which the present application is filed.

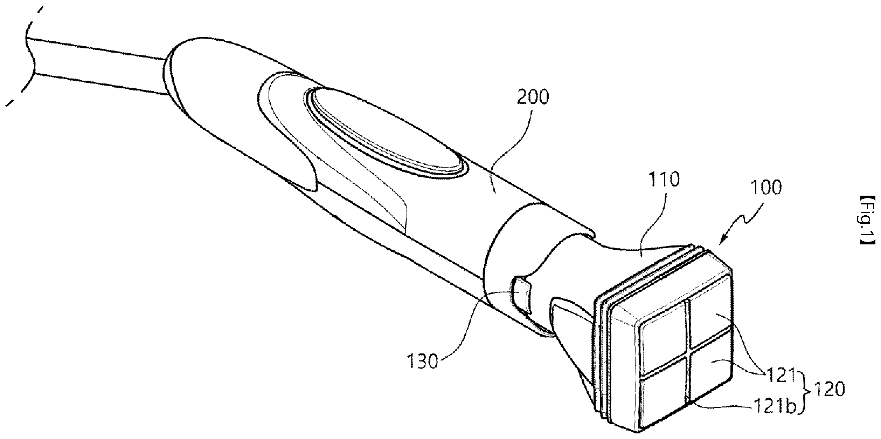

FIG. 1 is an exploded perspective view illustrating an embodiment of a handpiece for skin cosmetic treatment according to the present disclosure. An embodiment of a handpiece for skin cosmetic treatment according to the present disclosure includes an RF electrode cartridge including an RF electrode body 120 configured to be brought into contact with the skin, and includes a handpiece body casing 200 to which the RF electrode cartridge is coupled such that the RF electrode cartridge is capable of being detached from the handpiece body casing 200 and which is connected to a control body.

According to the present disclosure, an embodiment of the handpiece for skin cosmetic treatment provided with an RF electrode cartridge 100 including a relay for skin cosmetic treatment includes the RF electrode cartridge 100 including the RF electrode body 120 configured to be brought into contact with the skin, and includes the handpiece body casing 200 to which the RF electrode cartridge 100 is coupled such that the RF electrode cartridge 100 is capable of being detached from the handpiece body casing 200 and which is connected to the control body.

The handpiece body casing 200 is connected to the control body by a connection cable 10, and the control body is a known configuration which includes an RF signal generating part configured to generate an RF signal by applying electric power and AC power to the RF electrode body 120 and which is capable of being implemented with various modifications thereof. Accordingly, it should be noted that a more detailed description will be omitted for the control body.

The electrode body 120 includes a plurality of electrode members, and is configured to selectively apply electric power to the plurality of electrode members so that efficient treatment is capable of being realized according to the type of skin treatment and a skin condition of a patient.

That is, in the control body (not illustrated), a plurality of treatment modes in which electric power is selectively applied to the plurality of electrode members and the electric power is applied differently to the plurality of electrode members is provided. Therefore, when an operator selects any one of the plurality of treatment modes, the electric power is capable of being applied to the plurality of electrode members in a predetermined operation order accordingly.

Meanwhile, in the handpiece for skin cosmetic treatment provided with the RF electrode cartridge 100 including the relay for skin cosmetic treatment according to the present disclosure, in a state in which the operator holds the handpiece with the hand and puts the RF electrode body 120 to be brought into contact with the skin, an AC power is applied to the RF electrode body 120 by using the RF signal generating part so that an RF signal is generated, so that skin treatment may be realized and a cosmetic effect may be induced by heating the dermis of the skin.

At this time, an RF electrode panel body (not illustrated) having an area larger than an area of the RF electrode body 120 may be positioned opposite the handpiece for skin cosmetic treatment provided with the RF electrode cartridge 100 including the relay for skin cosmetic treatment according to the present disclosure.

The RF electrode panel body is a well-known configuration in which an RF signal is applied and the dermis of the skin is heated so that the skin treatment may be realized and the cosmetic effect may be induced, so that a more detailed description will be omitted.

That is, in the handpiece for skin cosmetic treatment according to the present disclosure, in a state in which the RF electrode body 120 is brought into contact with the skin, the RF signal is applied to the skin and the tissue inside the skin is heated, so that contraction to collagen inside the lower dermis or wound healing reaction is induced, thereby being capable of performing skin remodeling/resurfacing, wrinkle removal, and treatment of sebaceous glands, hair follicle fatty tissues, and spider veins.

The RF electrode cartridge 100 is a consumable part having a lifespan, being not capable of being used when the lifespan expires due to the use of the RF electrode cartridge 100 for treatment for a predetermined time, and being replaced. Furthermore, the RF electrode cartridge 100 is coupled to the handpiece body casing 200 such that the RF electrode cartridge 100 is capable of being detached from the handpiece body casing 200.

A cartridge insertion part 200b to which a part of the RF electrode cartridge 100 is inserted and coupled is positioned on a tip end part of the handpiece body casing 200.

The RF electrode cartridge 100 is inserted inside the cartridge insertion part 200b and is coupled to a cartridge coupling block 210 positioned inside the cartridge insertion part 200b, and is locked by a cartridge locking part 130 at a final coupling position, so that the coupling is completed.

The cartridge locking part 130 is a button-type locking part positioned on both sides of a cartridge casing 110, and the cartridge locking part 130 uses a known configuration in which a wedge part holding and supporting the cartridge coupling block 210 is positioned on a button part that is elastically supported and the locking is released by pressing the button part, so that a detailed description will be omitted.

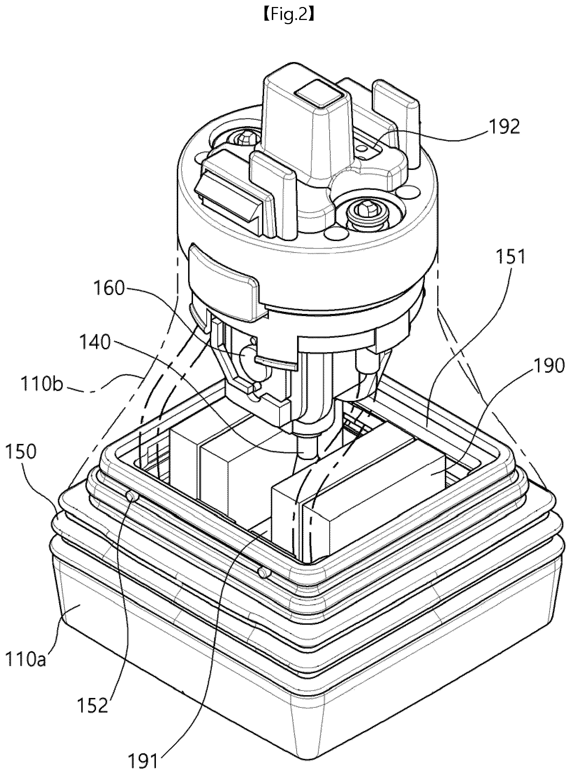

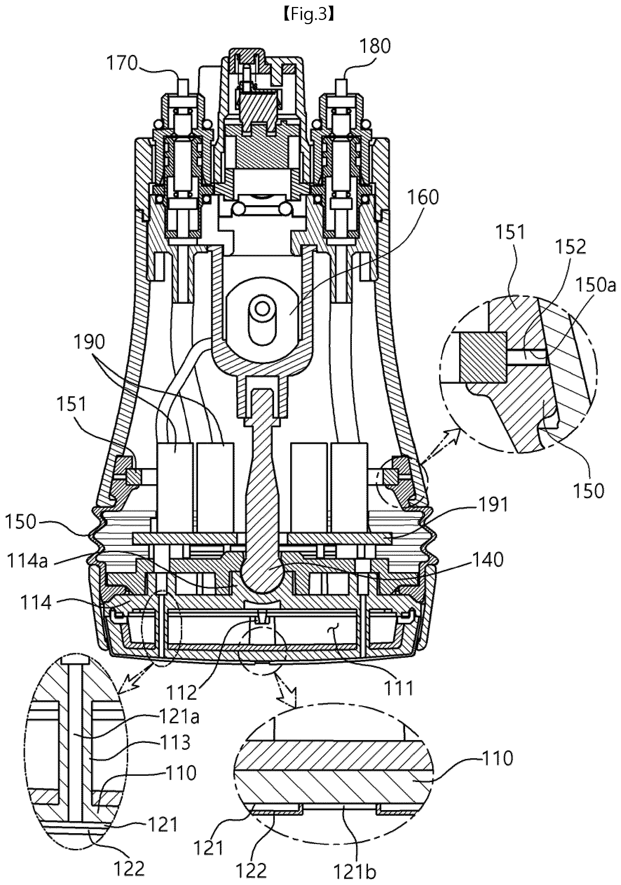

FIG. 2 is a perspective view illustrating an embodiment of the RF electrode cartridge including the relay for skin cosmetic treatment according to the present disclosure. More particularly, FIG. 2 is a perspective view illustrating an internal structure of the cartridge casing 110. FIG. 3 is a cross-sectional view illustrating an embodiment of the RF electrode cartridge including the relay for skin cosmetic treatment according to the present disclosure.

Referring to FIG. 2 and FIG. 3, an embodiment of the RF electrode cartridge including the relay for skin cosmetic treatment according to the present disclosure will be described in detail below.

According to the present disclosure, the RF electrode cartridge including the relay for skin cosmetic treatment includes the cartridge casing 110, and the RF electrode body 120 positioned on a first surface of the cartridge casing 110 and configured to be brought into contact with the skin, the RF electrode body 120 being configured to apply RF energy to the skin.

The RF electrode body 120 is positioned on an electrode mounting surface that is positioned at a first end portion of the cartridge casing 110.

It should be noted that the electrode mounting surface of the cartridge casing 110 is a surface facing the skin of the patient to be treated. That is, the electrode mounting surface of the cartridge casing 110 is a surface positioned at an end portion facing the skin when the operator holds the handpiece body casing 200 and performs the procedure.

The RF electrode body 120 may include an RF electrode member 121 positioned on the electrode mounting surface, formed of a conductor, and configured to apply RF energy to the inside of the skin, and may include an insulation film member 122 covering a surface of the RF electrode member 121.

The RF electrode body 120 may further include an electrode insulation member 121b provided such that the plurality of RF electrode members 121 is spaced apart from each other and is positioned on the electrode mounting space, the electrode insulation member 121b being positioned between the plurality of RF electrode member 121 spaced apart from each other, and the electrode insulation member 121b insulating the plurality of RF electrode members 121.

The electrode insulation member 121b insulates the plurality of RF electrode members 121 so that electric power is capable of being applied independently to each of the RF electrode members 121.

A height of the electrode insulation member 121b is formed lower than a height of the RF electrode member 121. That is, the electrode insulation member 121b is formed between the RF electrode members 121 such that the height of the electrode insulation member 121b is lower than the height of the RF electrode members 121, so that the plurality of RF electrode members 121 are stably brought into close contact with the skin of the patient.

The RF electrode member 121 is integrally attached to the electrode mounting surface such as a plating layer so that a structure formed integrally with the cartridge casing 110 and rigidity of the RF electrode member 121 is not changed, thereby being capable of minimizing the thickness of the RF electrode member 121.

In addition, as an example, the RF electrode member 121 is a plating layer formed integrally with the electrode mounting surface of the cartridge casing 110 by performing plating.

The RF electrode body 120 is formed such that a curved surface which is convex downward and which is not deformed when the RF electrode body 120 is pressed against the skin.

As an example, the RF electrode body 120 is formed such that the RF electrode body 120 has a surface curvature capable of maximizing an area of the RF electrode body 120 being brought into contact with the skin when the RF electrode 120 is pressed against the skin.

In addition, the RF electrode body 120 is formed in a shape in which a surface is convex. That is, the RF electrode body 120 is formed in a shape having uniform surface curvature 360-degree from a center thereof. Therefore, the shape is capable of evenly distributing a pressure force when the RF electrode body 120 presses the skin, and is capable of preventing pain from occurring when the skin is excessively pressed at an outer circumference edge of the RF electrode body 120.

The RF electrode body 120 is formed such that the RF electrode body 120 has the curved surface that does not deform when the RF electrode body 120 is pressed against the skin, so that the area of the RF electrode body 120 being brought into contact with the skin when the skin is pressed is maximized and the durability is secured since the RF electrode body 120 is not deformed during use.

The electrode mounting surface of the cartridge casing 110 is formed such that the electrode mounting surface has a curved surface which is convex downward and which is not deformed when the electrode mounting surface is pressed against the skin, so that the RF electrode member 121 formed by being plated on the surface of the electrode mounting surface is formed in the curved surface convex downward.

The RF electrode body 120 has the convex curved surface. Therefore, when the RF electrode body 120 is pressed with a small force during the procedure, the RF electrode body 120 is capable of being evenly brought into close contact with the skin that is being treated, so that convenience of the operator may be secured and also discomfort and pain of the patient during the procedure may be minimized.

A relay 190 electrically connected to the RF electrode body 120 and configured to control an operation of the RF electrode body 120 is positioned inside the cartridge casing 110.

In addition, the cartridge casing 110 is provided with a relay first connection terminal part 192 for electrically connecting the relay 190 to the control body.

As an example, the relay first connection terminal part 192 is positioned such that the relay first connection terminal part 192 is exposed to an upper surface of the cartridge casing 110. That is, the relay first connection terminal part 192 is positioned such that the relay first connection terminal part 192 is exposed to an upper surface of a second casing part 110b that will be described later.

As an example, the relay first connection terminal part 192 is electrically connected to a relay second connection terminal part 211 that is positioned inside the cartridge insertion part 200b of the handpiece body casing 200 that will be described later, and is electrically connected to the control body, which will be described in more detail in the following description of an embodiment of the handpiece for skin cosmetic treatment according to the present disclosure.

An electrode control substrate part 191 on which the relay 190 is mounted is positioned in the cartridge casing 110, and the electrode control substrate part 191 is electrically connected to the relay first connection terminal part 192 through a wire or a cable.

The RF electrode cartridge including the relay for skin cosmetic treatment according to the present disclosure is a consumable part that is replaced when the number of uses by the RF electrode body 120 reaches the preset number of uses. That is, the number of uses is the number of treatments, which is preset, and the RF electrode cartridge is required to be replaced when the RF electrode cartridge is used and the number of uses reaches the preset number.

In addition, the relay 190 is a consumable part that has the number of uses larger than the number of uses of the RF electrode body 120, but has a preset number of uses.

The RF electrode body 120 may have the plurality of electrode members, and may include a plurality of relays 190 accordingly.

The relay 190 is provided in a number corresponding to the number of electrode members, so that the electric power applied to each of the electrode members may be individually controlled.

The RF electrode cartridge including the relay for skin cosmetic treatment according to the present disclosure is replaced after being used for the preset number of uses. At this time, the relay 190 is also replaced.

In a situation in which the relay 190 is positioned in the handpiece body casing 200, when the number of uses of the relay 190 reaches the preset number of uses separately from the number of uses of the RF electrode cartridge, the handpiece body casing 200 is required to be replaced. Therefore, a high cost is required, and a hassle of replacing the handpiece body casing 200 and an inconvenience of not being capable of using the device occur.

That is, in the RF electrode cartridge including the relay for skin cosmetic treatment according to the present disclosure, the relay 190 having the preset number of uses larger than the preset number of uses of the RF electrode body 120 is included so that the relay 190 is also replaced when the RF electrode body 120 is replaced, so that the handpiece body casing 200 is not required to be replaced compared to when the relay 190 is positioned in the handpiece body casing 200.

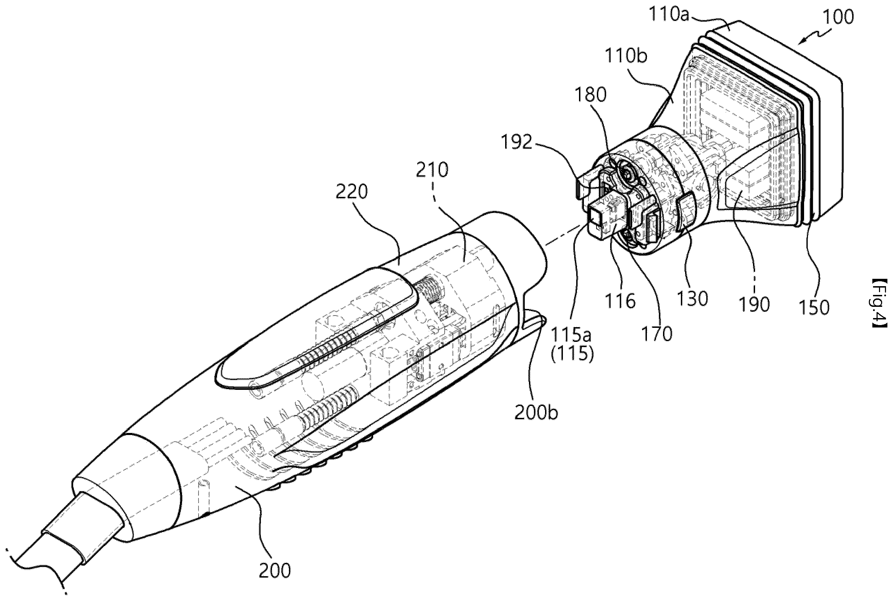

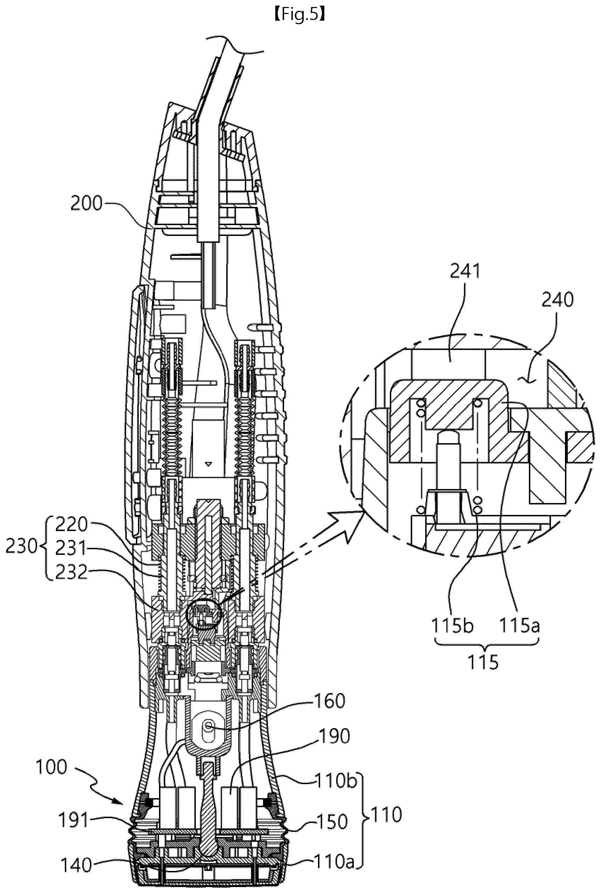

Meanwhile, FIG. 4 is an exploded perspective view illustrating an embodiment of the handpiece for skin cosmetic treatment according to the present disclosure, and FIG. 5 is a cross-sectional view illustrating an embodiment of the handpiece for skin cosmetic treatment according to the present disclosure.

Referring to FIG. 3 to FIG. 5, an embodiment of the RF electrode cartridge including the relay for skin cosmetic treatment according to the present disclosure will be described in detail below.

The cartridge casing 110 includes a first casing part 110a in which the RF electrode body 120 is positioned on a first surface of the first casing part 110a, and includes a second casing part 110b to which the first casing part 110a is connected such that the first casing part 110a is capable of being tilted. Therefore, the cartridge casing 110 is tilted when the RF electrode body 120 is brought into contact with the skin and the RF electrode body 120 is moved, so that a state in which the RF electrode body 120 is brought into contact with the skin is capable of being maintained.

The second casing part 110b is a part coupled to the handpiece body casing 200 such that the second casing part 110b is capable of being detached from the handpiece body casing 200, and the second casing part 110b has a first end portion provided with a casing coupling part which is inserted inside the cartridge insertion part 200b of the handpiece body casing 200 and which is coupled to the cartridge coupling block 210.

In addition, the first casing part 110a is connected to a second end portion of the second casing part 110b such that the first casing part 110a is capable of being tilted.

More specifically, the RF electrode cartridge 100 including the relay for skin cosmetic treatment according to the present disclosure further includes a ball joint part 140 connecting the first casing part 110a and the second casing part 110b to each other such that the first casing part 110a is capable of being freely tilted at a 360-degree radius.

For example, the ball joint part 140 includes a joint support bar member fixed to the second end portion of the second casing part 110b, and includes a ball body positioned at an end portion of the j oint support bar member and coupled to the first casing part 110a such that the ball body is capable of being rotated.

In the first casing part 110a, a ball insertion part is positioned such that the ball body is inserted into the ball insertion part and the ball body is capable of being rotated, and the first casing part 110a is capable of being freely tilted at the 360-degree radius around the ball body.

In addition, an embodiment of the RF electrode cartridge 100 including the relay for skin cosmetic treatment according to the present disclosure may further include a bellows member 150 having a first end portion connected to the first casing part 110a, having a second end portion connected to the second casing part 110b, being positioned so as to surround the ball joint part 140, and being provided with a plurality of corrugated parts.

In the bellows member 150, the plurality of corrugated parts is positioned in a longitudinal direction of the handpiece for skin cosmetic treatment, and supports a tilting movement of the first casing part 110a by being freely folded and unfolded.

The bellows member 150 has the first end portion fixed along an outer circumference of the first casing part 110a, and has the second end portion fixed along an outer circumference of the second casing part 110b, thereby surrounding an outside of the centrally positioned ball joint part 140 at a position spaced apart from the ball joint part 140.

The bellows member 150 surrounds the outside of the ball joint part 140 at a position spaced apart from the ball joint part 140, so that the first casing part 110a is capable of being freely tilted at the 360-degree radius around the ball body. Furthermore, the bellow member 150 covers a space between the first casing part 110a and the second casing part 110b, thereby preventing foreign substances from being introduced into the first casing part 110a and the second casing part 110b.

In addition, an embodiment of the RF electrode cartridge 100 including the relay for skin cosmetic treatment according to the present disclosure further includes a bellows supporting frame member 151 positioned at an upper end portion of the bellows member 150 and formed in a frame structure that surrounds an inner circumference of the bellows member 150.

For example, the bellows supporting frame member 151 has the frame structure surrounding the inner circumference of the bellows member 150, forming a space where the ball joint part can pass therethrough, and having a rectangular frame structure. Furthermore, it should be noted that the bellows supporting frame member 151 may be modified and implemented in various frame shapes according to the shape of the bellows member 150. That is, the bellows supporting frame member 151 may be modified and implemented in various frame shapes according to the shape of the connection portion between the first casing part 110a and the second casing part 110b.

In addition, in the bellows supporting frame member 151, a plurality of frame fixing pins 152 protrudes on an outer side surface of the bellows supporting frame member 151, and a plurality of pin insertion parts 150a into which the plurality of frame fixing pins 152 is inserted is provided in the bellows member 150.

The frame fixing pins 152 protrude on each outer side surface of the respective bellows supporting frame members 151, and are inserted and coupled to the respective pin insertion parts 150a of the bellows member 150, so that positions of the bellows supporting frame members 151 may be stably fixed.

The bellows supporting frame member 151 is positioned on the upper end portion of the bellows member 150 connected to the second casing part 110b, so that the rigidity of the connection portion of the bellows member 150 may be increased and an accident in which the bellows member 150 is deformed or damaged at the connection portion between the second casing part 110b and the bellows member 150 may be prevented.

In addition, an embodiment of the RF electrode cartridge 100 including the relay for skin cosmetic treatment according to the present disclosure may further include a vibration motor unit 160 positioned inside the cartridge casing 110.

The vibration motor unit 160 is positioned on a ball j oint supporting part to which the ball joint part is connected, and is configured to generate vibration during the procedure so that a massaging effect is applied to the skin being brought into close contact with the RF electrode body 120, thereby being capable of further increasing a treatment effect and also being capable of reducing pain caused to the patient by electrical stimulation during the treatment.

A cooling space 111 in which a coolant that cools the RF electrode body 120 is filled is positioned inside the cartridge casing 110. Furthermore, the RF electrode cartridge 100 including the relay for skin cosmetic treatment according to the present disclosure includes a first coolant line part 170 and a second coolant line part 180 which are configured to supply the coolant to an inside of the cooling space and to discharge the coolant inside the cooling space 111 such that the coolant is circulated.

The cooling space 111 is positioned in the first casing part 110a in which the RF electrode body 120 is positioned, and includes the electrode mounting surface.

The first coolant line part 170 and the second coolant line part 180 are connected to a coolant circulation line positioned in the handpiece for skin cosmetic treatment when the RF electrode cartridge 100 including the relay for skin cosmetic treatment according to the present disclosure is coupled to the handpiece for skin cosmetic treatment.

The first coolant line part 170 and the second coolant line part 180 are positioned such that the first coolant line part 170 and the second coolant line part 180 protrude toward an upper portion of the cartridge casing 110. Therefore, when the casing coupling part is inserted inside the cartridge insertion part 200b and the casing coupling part is coupled to the cartridge coupling block 210, the first coolant line part 170 and the second coolant line part 180 are connected to a coolant circulation line part 200a of the handpiece body casing 200.

The coolant circulation line part 200a is connected to a coolant tank positioned in the control body. The first coolant line part 170 and the second coolant line part 180 are connected to the coolant tank positioned in the control body through the coolant circulation line part 200a.

The coolant passes through the coolant tank and the cooling space 111 and is circulated through the coolant circulation line part 200a, the first coolant line part 170, and the second coolant line part 180 by an operation of a pump. Furthermore, during the circulation, a temperature of the coolant may be maintained at a constant temperature in the cooling space 111 by being cooled by a cooling part positioned on a coolant circulation line or the coolant tank.

The cooling part may include a known cooling device such as a radiator or a chiller that cools a coolant.

In addition, a temperature sensor 112 configured to detect a temperature of the coolant is positioned in the cooling space 111, and the temperature sensor detects the temperature of the temperature and transmit temperature information to a control unit in the control body.

The control unit is configured to control an operation of the pump and the cooling part by using the coolant temperature information transmitted from the temperature sensor. Therefore, the temperature of the coolant is capable of being maintained at a temperature suitable for cooling the skin during the treatment.

As an example, the temperature sensor 112 is positioned in the cooling space 111, is configured to measure the temperature of the coolant substantially used for cooling the skin, and is configured to maintain the temperature of the coolant at the temperature suitable for cooling the skin. Furthermore, it should be noted that the temperature sensor 112 may be implemented such that the temperature sensor 112 is capable of being positioned in any position such as the coolant circulation line or the coolant tank where the temperature sensor 112 is capable of measuring the temperature of the coolant.

After the coolant is discharged from the inside of the cooling space 111 by the operation of the pump, the coolant is cooled by the cooling part while passing through the coolant tank, and then is introduced into the cooling space 111 again, thereby being capable of cooling the surface of the skin when the skin is heated due to RF signal application.

That is, since the temperature of the RF electrode member 121 that is heated during the procedure is lowered by the circulating coolant, the surface of the skin being treated is cooled, so that damage caused by burns and heat of the skin may be prevented during the treatment.

The RF electrode cartridge 100 including the relay for skin cosmetic treatment according to the present disclosure is capable of continuously and stably cooling the skin with the circulating coolant in the cooling space 111, and is capable of cooling the skin at an even and constant temperature, so that discomfort caused by skin cooling may be minimized and satisfaction with the treatment may be increased during the skin treatment.

Meanwhile, the RF electrode member 121 may be provided with an electrode connection terminal 121a which protrudes upward and which is connected to the electrode control substrate part 191 so that the electrode connection terminal 121a is electrically connected to the RF signal generating part of the control body.

The electrode connection terminal 121a is connected to the electrode control substrate part 191, and the electrode control substrate part 191 is electrically connected to the relay first connection terminal part 192 through a wire or a cable.

The electrode connection terminal 121a is configured to generate the RF signal by applying AC power to the RF electrode member 121.

As an example, the electrode connection terminal 121a is provided such that a plurality of electrode connection terminals 121a is positioned corresponding to the plurality of RF electrode members 121, and a lower end portion of the electrode connection terminal 121a is connected to the RF electrode member 121 by passing through the cartridge casing 110. That is, the lower end portion of the electrode connection terminal 121a is connected to the RF electrode member 121 by passing through the first casing part 110a.

In addition, the electrode connection terminal 121a passes through the cooling space 111 and is connected to the electrode control substrate part 191 by penetrating an upper surface of the cooling space 111. That is, the electrode connection terminal 121a penetrates a cooling space cover part 114.

In addition, the electrode connection terminal 121a may be connected to the relay first connection terminal part 192 through the electrode control substrate part 191, and may be electrically connected to the control body through the relay first connection terminal part 192.

A terminal passage through which the electrode connection terminal 121a passes is provided in the cooling space 111, and a terminal insulation part 113 that insulates the electrode connection terminal 121a from the coolant in the cooling space 111 is positioned in the cooling space 111.

The cooling space 111 has a structure in which an inner portion of the cooling space 111 is sealed since the cooling space cover part 114 on which a ball coupling part 114a that protrudes and is coupled to the ball joint is positioned is positioned on an upper portion of the cooling space 111.

In a structure in which the cooling space 111 is sealed since the cooling space 111 is integrally connected to the cooling space cover part 114 which protrudes on a bottom surface of the cooling space 111 as an integral manner and which has an upper end portion sealing the upper portion of the cooling space 111, the terminal insulation part 113 is configured such that the electrode connection terminal 121a penetrates the cooling space 111 and is capable of being electrically connected to the control body from outside the cooling space 111 while the electrode connection terminal 121a is insulated from the coolant.

In the present disclosure, the RF electrode body 120 is formed as an integrated plating layer on a lower surface of an electrode supporting body, so that a manufacturing process may be simplified and a manufacturing cost may be reduced, thereby being capable of securing economic efficiency.

In addition, in the present disclosure, the durability of the RF electrode body 120 is secured since the RF electrode body 120 has a fixed shape in which the RF electrode body 120 is not deformed when the RF electrode body 120 is pressed by the skin.

In addition, in the present disclosure, by circulating the coolant, a skin surface temperature may be maintained at a constant temperature by cooling the surface of the skin that is heated by the RF signal. Therefore, treatment efficiency may be increased, discomfort caused by skin cooling during the treatment may be minimized, and satisfaction during the treatment may be increased.

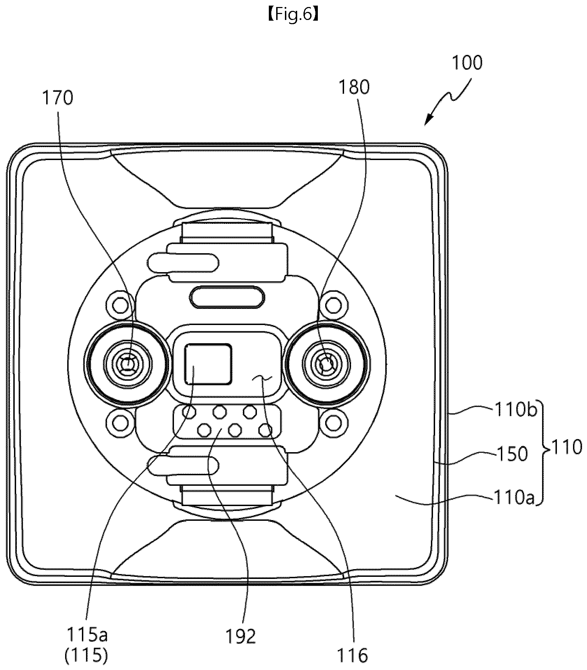

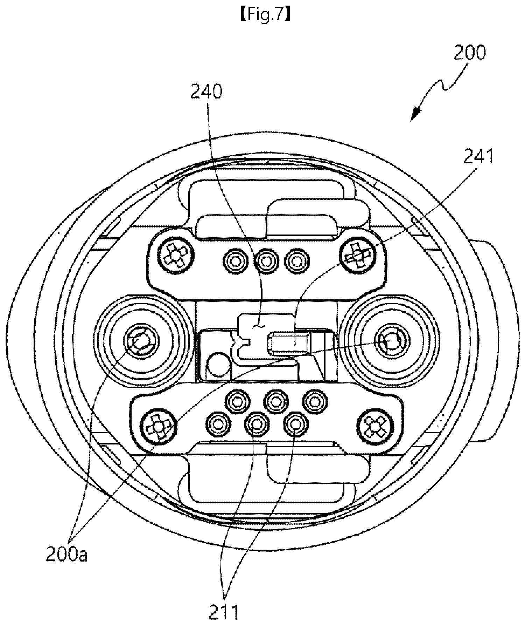

Meanwhile, FIG. 6 is a plan view illustrating the RF electrode cartridge for skin cosmetic treatment in an embodiment of the handpiece for skin cosmetic treatment according to the present disclosure, and FIG. 7 is a bottom view illustrating the handpiece body casing 200 in an embodiment of the handpiece for skin cosmetic treatment according to the present disclosure.

Referring to FIG. 4 to FIG. 7, an embodiment of the handpiece for skin cosmetic treatment according to the present disclosure will be described in detail below.

The relay second connection terminal part 211 to which the relay first connection terminal part 192 is connected is positioned in the cartridge insertion part 200b that is positioned at the tip end portion of the handpiece body casing 200, and the cartridge coupling block 210 where a line connection part 212 connecting the first coolant line part 170 and the second coolant line part 180 to the coolant circulation line part 200a is positioned is positioned in the cartridge insertion part 200b such that the cartridge coupling block 210 is capable of being moved.

The relay first connection terminal part 192 is electrically connected to the control body through the relay second connection terminal part 211 and the connection cable 10.

When the RF cartridge is inserted into the cartridge insertion part 200b, the relay first connection terminal part 192 is connected to the relay second connection terminal part 211 of the cartridge coupling block 210, and the first coolant line part 170 and the second coolant line part 180 are inserted and fitted into the line connection part 212 of the cartridge coupling block 210, so that the first coolant line part 170 and the second coolant line part 180 are connected to the coolant circulation line part 200a.

In addition, the cartridge coupling block 210 is positioned such that the cartridge coupling block 210 is capable of being moved in the longitudinal direction of the handpiece body casing 200, and a spring member 220 for a cartridge configured to elastically support the cartridge coupling block 210 is positioned in the handpiece body casing 200.

The coolant circulation line part 200a may be a hose which is capable of being freely bent within the handpiece body casing 200 and which has a spare length equal to at least a movement distance of the RF electrode cartridge 100, or may be a hose which has a bellows structure and which is configured such that a length of the hose is capable of being adjusted within the handpiece body casing 200.

The spring member 220 for the cartridge is configured to elastically support the cartridge coupling block 210 to which the RF electrode cartridge 100 is coupled and which is capable of being moved, so that the spring member 220 for the cartridge serves to buffer a load applied to the patient when the RF electrode body 120 is pressed and is brought into close contact with the skin of the patient. Furthermore, the spring member 220 is configured to push the RF electrode cartridge 100 toward the skin of the patient with an elastic resilience force so that the RF electrode body 120 is more fully brought into close contact with the skin of the patient.

A cartridge movement guide part 230 configured to guide a rectilinear movement of the cartridge coupling block 210 is positioned in the handpiece body casing 200.

The cartridge movement guide part 230 may include a movement guide bar part 231 that is positioned such that the movement guide bar part 231 protrudes on the cartridge coupling block 210, and may include a movement guide block 232 which is positioned in the handpiece body casing 200 and through which the movement guide bar part 231 passes and the movement guide bar part 231 is movably coupled to the movement guide block 232.

In addition, as an example, the movement guide bar part 231 includes a plurality of movement guide bar parts 231, and at least one of the plurality of movement guide bar parts 231 is positioned through the spring member 220 for the cartridge, the spring member 220 being a coil spring.

That is, as an example, the spring member 220 for the cartridge is the coil spring, and the movement guide bar part 231 is positioned by penetrating the spring member 220 for the cartridge such that a first end portion of the movement guide bar part 231 is supported on the cartridge insertion part 200b and a second end portion of the movement guide bar part 231 is supported on the movement guide block 232, so that the spring member 220 for the cartridge elastically supports the rectilinear movements of the cartridge coupling block 210 and the RF electrode cartridge 100 coupled to the cartridge coupling block 210.

Meanwhile, in the RF electrode cartridge 100 including the relay for skin cosmetic treatment according to the present disclosure, an electrode on/off switch 115 configured to turn on/off electric power applied to the RF electrode body 120 is positioned on the upper surface of the cartridge casing 110. That is, the electrode on/off switch 115 is positioned on the upper surface of the second casing part 200b.

The electrode on/off switch 115 includes a switch button 115a elastically supported by a switch spring 115b, and has a structure in which electric power applied to the RF electrode body 120 is turned on only when the switch button 115a is pressed and pushed and the electric power is turned off when the switch button 115a is released from a pressed state.

In addition, in the inner portion of the handpiece body casing 200, which is the inner portion of the cartridge insertion part 200b, a protrusion part 241 for pressing a switch configured to press and push the electrode on/off switch 115 when the cartridge coupling block 210 is pushed and moved is positioned such that the protrusion part 241 for pressing the switch protrudes.

The protrusion part 241 for pressing the switch is positioned such that the protrusion part 241 for pressing the switch is in contact with an upper surface of the switch button 115a in a state in which the RF electrode cartridge 100 is coupled to the handpiece body casing 200.

More specifically, on the upper surface of the cartridge casing 110, which is the upper surface of the second casing part 200b, a switch mounting protrusion part 116 having an upper surface on which the electrode on/off switch is positioned is positioned such that the switch mounting protrusion part 116 protrudes. Furthermore, a protrusion insertion part 240 into which the switch mounting protrusion part 116 is inserted is positioned inside the cartridge insertion part 200b, and the protrusion part 241 for pressing the switch protrudes and is positioned in the protrusion insertion part 240 such that the protrusion part 241 for pressing the switch faces the switch mounting protrusion part 116.

The electrode on/off switch 115 is positioned such that the upper surface of the switch button 115a protrudes toward an upper surface of the switch mounting protrusion part 116 or the upper surface of the switch button 115a is in a plane with the upper surface of the switch mounting protrusion part 116. When the RF electrode cartridge 100 is coupled to the handpiece body casing 200, the switch mounting protrusion part 116 is inserted inside the protrusion insertion part 240, and the protrusion part 241 for pressing the switch is positioned such that the protrusion part 241 for pressing is in contact with the upper surface of the switch button 115a.

That is, in a state in which the operator holds the handpiece body casing 200 and the RF electrode body 120 is brought into close contact with the skin, when the operator pushes the handpiece body casing 200, the RF electrode cartridge 100 is pushed and is moved toward the handpiece body casing 200, and the cartridge coupling block 210 is pushed and moved, so that the switch mounting protrusion part 116 pushes the switch button 115a, thereby applying electric power to the RF electrode body 120.

In addition, in a state in which the RF electrode body 120 is brought into contact with the skin, when the handpiece body casing 200 is not pushed, which is when the operator does not push the handpiece body casing 200, the cartridge coupling block 210 is moved to the original position by the spring member 220 for the cartridge, and the switch button 115a is also returned to the original position by the elastic resilience force of the switch spring 115b, so that electric power applied to the RF electrode body 120 is blocked and the operation of the RF electrode body 120 is turned off.

In the handpiece for skin cosmetic treatment according to the present disclosure, electric power is applied to the RF electrode body 120 only when the operator holds and pushes the handpiece body casing 200 during the procedure. Furthermore, when the operator releases the force pushing the handpiece body casing 200 during the procedure, the electric power applied to the RF electrode body 120 is automatically blocked due to the elastic resilience force of the spring. Therefore, the procedure is capable of being accurately performed in the required area and the exact number of uses, and an accident such as skin damage due to excessive stimulation during the treatment is also capable of being prevented.

In the RF electrode cartridge 100 including the relay for skin cosmetic treatment according to the present disclosure and the handpiece including the RF electrode cartridge 100 for skin cosmetic treatment, the procedure is performed by moving the RF electrode body 120 while the RF electrode body 120 is brought into close contact with the skin of the patient.

At this time, the first casing part 110a of the RF electrode cartridge 100 is configured to be tilted freely at the 360-degree radius around the ball joint part 140, so that the RF the state in which the RF electrode body 120 is continuously in close contact with the skin may be maintained.

In addition, the massaging effect is generated on the skin by vibration caused by the vibration motor unit, thereby further inducing a treatment effect according to the procedure.

In addition, the pressing force caused by the operator is buffered by the elastic supporting of the spring member 220 for the cartridge, and the state in which the RF electrode part 120 is brought into close contact with the skin may be more reliably maintained due to the elastic resilience force of the spring member 220 for the cartridge.

In the present disclosure, during the procedure, since the RF electrode body 120 is tilted freely at the 360-degree radius around the ball joint part 140 and is moved while being brought into close contact with the skin, an accident in which the RF electrode body 120 is separated from the skin and a damage to the skin tissue or a burn to the skin occurs is prevented from occurring, so that safety during the procedure may be significantly increased.

In the present disclosure, the relay 190 that controls the electrical signal applied to the RF electrode is positioned in the RF electrode cartridge, so that the relay 190 that is the consumable part is replaced when the RF electrode cartridge is replaced. Therefore, the handpiece body casing 200 is capable of being used without being replaced, so that the hassle and the cost of replacing the handpiece body casing 200 may be reduced.

In the present disclosure, since the vibration motor unit is provided, the pain that occurs during the skin treatment may be minimized, and the treatment effect may be increased.

In the present disclosure, since electric power is applied to the RF electrode body 120 only when the RF electrode cartridge that is brought into contact with the skin is pressed, a safe and convenient treatment is capable of being realized, and safety during the treatment may be increased.

It is to be understood that the present disclosure is not limited to the above described embodiments but may be variously modified and embodied within the scope of the present disclosure without departing from the gist of the present disclosure.

## Claims

1. An RF electrode cartridge comprising a relay for skin cosmetic treatment, the RF electrode cartridge comprising:
a cartridge casing;
an RF electrode body positioned on a first surface of the cartridge casing, the RF electrode body being brought into contact with skin, and the RF electrode body being configured to apply RF energy inside the skin;
the relay mounted in the cartridge casing and electrically connected to the RF electrode body, the relay being configured to control an operation of the RF electrode body; and
a relay first connection terminal part for electrically connecting the relay to a control body.

2. The RF electrode cartridge of claim 1, wherein the RF electrode body comprises:
a plurality of RF electrode members spaced apart from each other and positioned on the first surface of the cartridge casing; and
an electrode insulation member positioned between the plurality of RF electrode members spaced apart from each other, the electrode insulation member insulating the plurality of RF electrode members.

3. The RF electrode cartridge of claim 2, wherein a height of the electrode insulation member is formed lower than each height of the plurality of RF electrode members.

4. The RF electrode cartridge of claim 1, wherein an electrode control substrate part on which the relay is mounted is positioned in the cartridge casing, and the electrode control substrate part is electrically connected to the relay first connection terminal part through a wire or a cable.

5. The RF electrode cartridge of claim 1, wherein the RF electrode body comprises a plurality of RF electrode members spaced apart from each other and positioned on the first surface of the cartridge casing, and the relay is provided in a number corresponding to the number of RF electrode members so that the relay individually controls electric power applied to each of the RF electrode members.

6. The RF electrode cartridge of claim 1, wherein the cartridge casing comprises:
a first casing part in which the RF electrode body is positioned on a first surface the first casing part;
a second casing part to which the first casing part is coupled such that the first casing part is capable of being tilted;
a ball j oint part connecting the first casing part and the second casing part to each other so that the first casing part is capable of being tilted freely at a 360-degree radius;
a bellows member having a first end portion connected to the first casing part and having a second end portion connected to the second casing part, the bellows member being positioned such that the bellows member surrounds the ball j oint part, and the bellows member being provided with a plurality of corrugated parts; and
a bellows supporting frame member positioned at an upper end portion of the bellows member and formed in a frame structure surrounding an inner circumference of the bellows member.

7. The RF electrode cartridge of claim 6, wherein a plurality of frame fixing pins protrudes on an outer side surface of the bellows supporting frame member, and a plurality of pin insertion parts into which the plurality of frame fixing pins is inserted is provided in the bellows member.

8. The RF electrode cartridge of claim 1, further comprising a vibration motor unit positioned in the cartridge casing.

9. A handpiece for skin cosmetic treatment, the handpiece comprising:
an RF electrode cartridge comprising an RF electrode body being brought into contact with skin; and
a handpiece body casing to which the RF electrode cartridge is coupled such that the RF electrode cartridge is capable of being detached from the handpiece body casing, the handpiece body casing being connected to a control body,
wherein the RF electrode cartridge comprises the RF electrode cartridge comprising the relay for skin cosmetic treatment of any one of claim 1 to claim 8.

10. The handpiece of claim 9, wherein a cartridge insertion part to which the RF electrode cartridge is inserted and coupled is positioned on a tip end portion of the handpiece body casing,
a cartridge coupling block to which the RF electrode cartridge is coupled so that the cartridge coupling block electrically connects the control body and the RF electrode body to each other is positioned in the cartridge insertion part such that the cartridge coupling block is capable of being moved,
the cartridge coupling block is positioned in a state in which the cartridge coupling block is elastically supported by a spring member for a cartridge,
an electrode on/off switch configured to turn on/off electric power applied to the RF electrode body is positioned on an upper surface of a cartridge casing,
the electrode on/off switch comprises a switch button elastically supported by a switch spring, and has a structure in which the electric power applied to the RF electrode body is turned on only when the switch button is in a state in which the switch button is pressed and pushed, and the electric power is turned off when the state in which the switch button is pressed is released, and
a protrusion part for pressing a switch protrudes and is positioned inside the cartridge insertion part, and the protrusion part is configured to press and push the electrode on/off switch when the cartridge coupling block is pushed and moved inward.

11. The handpiece of claim 10, wherein a switch mounting protrusion part having an upper portion on which the electrode on/off switch is positioned protrudes and is positioned on the upper surface of the cartridge casing, and a protrusion insertion part into which the switch mounting protrusion part is inserted is positioned inside the cartridge insertion part, and
the protrusion part for pressing the switch is positioned in the protrusion insertion part such that the protrusion part for pressing the switch faces an upper surface of the switch mounting protrusion part.
